# EUROPEAN PATENT APPLICATION

(11) **EP 4 843 481 A2**
(43) Date of publication of application: **25.11.2026**
(21) Application number: 26202371.6
(22) Date of filing: 21.06.2022
(51) Int. Cl.: A61B 5/00

(54) **SYSTEM AND METHOD OF VOICE BIOMARKER DISCOVERY FOR MEDICAL DIAGNOSIS USING NEURAL NETWORKS**

(30) Priority: 22.06.2021 US 202163213356 P
(62) Document number(s) of the earlier application(s) in accordance with Art. 76 EPC: 22829486.4 / 4 360 086
(71) Applicant: AIVA Global, Inc., Boothwyn, PA 19061 (US)
(72) Inventor: SINGH, Rita, Pittsburgh, 15217 (US)
(74) Representative: K&L Gates LLP

(57) **Abstract**

A computer system comprising one or more processor cores and a memory in communication with the processor cores. The memory stores software that when executed by the one or more processor cores, causes the one or more processing cores to train a neural network system to generate a biomarker that is discriminative for a target medical condition from a voice recording of a subject person and to detect whether the subject person has the target medical condition based on the voice recording of the subject person. The neural network system is trained by training an autoencoder with training voice recordings and training a first classifier to detect the target medical condition. The autoencoder comprises an encoder and a decoder. The encoder is trained to generate a latent feature representation, and the decoder is trained to transform the latent feature representation to an output feature stack.

## Description

### PRIORITY CLAIM

The present application claims priority to United States provisional application Serial No. 63/213,356, filed June 22, 2021, titled "System and Method of Voice Biomarker Discovery for Medical Diagnosis Using Neural Networks," which is incorporated herein by reference.

### BACKGROUND

A biomarker for a medical condition is an objectively specifiable vector or tensor that corresponds to a pattern in one or more multidimensional mathematical spaces (which may or may not be human interpretable or viewable in their entirety), such that it is highly discriminative for that condition in any computational setting. Here the term "medical condition" includes, but is not limited to factors and parameters that are relevant to the human body and its correct functioning, such as diseases, syndromes, infections, physical and physiological abnormalities, etc. Voice is known to carry biomarkers for multiple medical conditions, but it is hard to measure them objectively even for those conditions for which biomarkers have been observed to exist. For other conditions that have biological pathways to the human vocal production mechanism, biomarkers can be hypothesized to be present in voice, even if they are not human-observable (i.e. they may be imperceptible).

A number of biomarkers and signal features related to biomarkers have already been identified in the scientific literature. These include, but are not limited to spectra, spectrographic representations, voicing-onset time, formants, formant bandwidth, modulation, harmonicity, fundamental frequency and its harmonics, jitter, shimmer, resonances and antiresonances, etc. These features, which may be directly derived from the raw signal, spectrographic time-frequency representations and other transform domains, are derived using various mathematically well-motivated digital signal processing (DSP) or other Machine Learned Signal Processing (MLSP) techniques, and may be viewed as the "measurable" properties of the voice signal. However, the set of such measurements is limited and enumerable, due to the limited number of digital signal processing (DSP) algorithms available to compute them, and by the time-frequency (and other) resolution tradeoffs implicit in them, and may not be sufficiently diverse, or of fine-enough resolution to capture all biomarkers relevant to a target medical condition.

Alternate approaches use neural networks to derive features from the incoming signal, to classify a medical condition. While this approach requires less expert knowledge of the target medical condition or its influence on the voice signal, it derives abstract, uninterpretable features, which may in turn lose information about the *measurable* properties of the signal that can be effectively derived by the more conventional signal-processing approach described earlier. In the absence of sufficiently large, diverse training data, it also remains uncertain whether these methods derive actual biomarkers for the target condition, or merely some incidental features that are specific to the training data or conditions used.

### SUMMARY

The systems and methods of the present invention enable neural-network based extraction of biomarkers from voice signal that retain the measurable properties of the signal captured by signal-processing approaches, while also potentially capturing information that is not captured by traditional signal processing approaches. This is done through the combination of an appropriately configured neural-network system to extract biomarkers from the voice signal, and ensuring that these biomarkers explicitly retain the measurable information derived using conventional signal processing methods, and while also remaining maximally discriminative for the target medical condition against other potentially confusable conditions.

In one general aspect, the present invention is directed to a neural network-based system that is trained through machine learning to discover one or many different voice biomarkers, and the one or more multidimensional mathematical spaces in which they exist. A method according to various embodiments of the present invention includes training, with a computer system comprising one or more processor cores, a machine learning system to discover a biomarker for a target medical condition from voice recording waveforms. The training voice recordings can be from one or more persons, with at least one person having the target medical condition. The training comprises, among other things: (i) a (digital or neural) signal processing stack that receives as input the voice recording waveform, performs a set of digital signal processing and (optionally) machine learning operations on it, and outputs a set of biomarker-relevant measurements; (ii) an encoder that receives as input a set of feature values, wherein the set of features values are obtained from performing the digital signal processing on the voice recording waveform, and wherein the output of the encoder is a latent feature representation; (iii) a decoder to transform the latent feature representation output by the encoder back either to a waveform, or to some intermediate representation from which are derived, using appropriate signal processing, a feature stack that approximates the set of feature values input to the encoder, and a set of quantitative biomarker-related measurements that approximate the output of the DSP/neural signal processing stack; (iv) a classifier or predictor targeted at the target medical condition which receives as input the latent representation and outputs a categorical or numeric prediction of the target condition; and (v) a validation stack comprising a collection of classifiers or predictors for medical conditions, where the conditions are different from the target medical condition and may be confusable with it, and which receives as input the latent representation, and outputs classification or numeric predictions that closely match the true values of these conditions. The inter-connected neural network subsystems, including the encoder and decoder, may be trained with a global, system-wide loss function, so that the various neural network subsystems are trained with a collective objective (in addition to local objectives that each neural network is trained with). The result of the simultaneously global and local objective training for the neural networks is the biomarker latent space, which can be used for many purposes, such as training machine learning classifiers to detect the condition corresponding to the biomarker in voice recordings.

### FIGURES

Various embodiments of the present invention are described herein by way of example in conjunction with the following figures.
Figures 1 and 2 collectively illustrate a neural network-based system for discovering a voice biomarker according to various embodiments of the present invention.
Figure 3 depicts a feedforward neural network.
Figure 4 depicts a computer system according to various embodiments of the present invention.

### DESCRIPTION

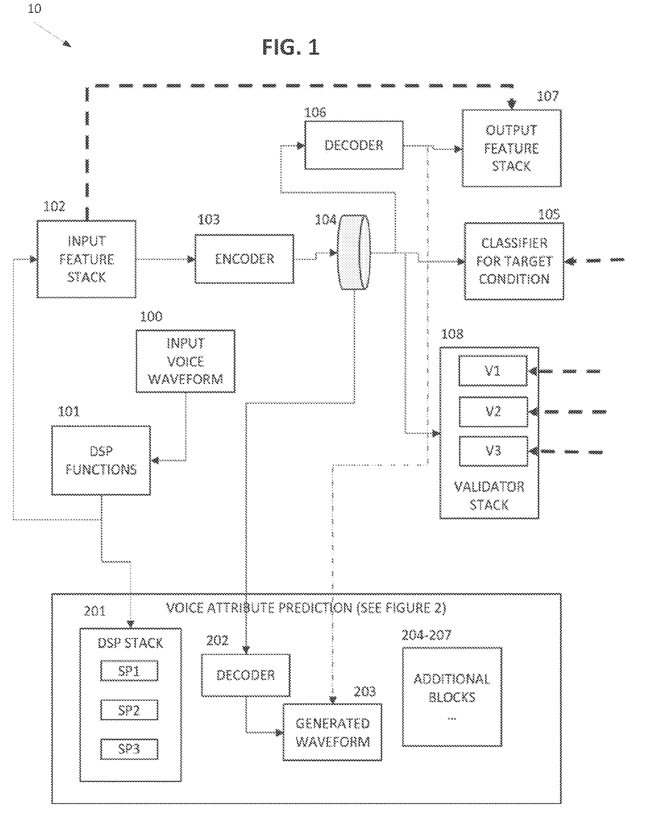

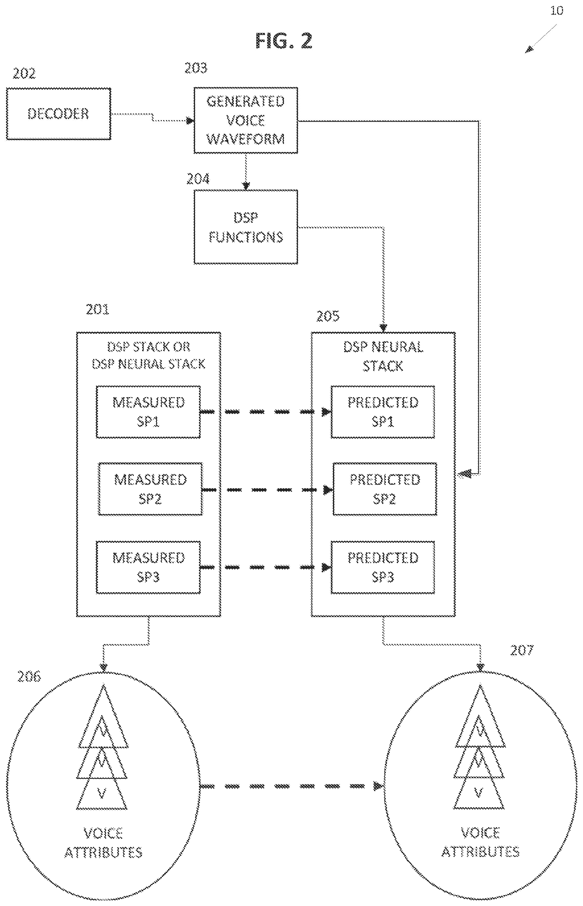

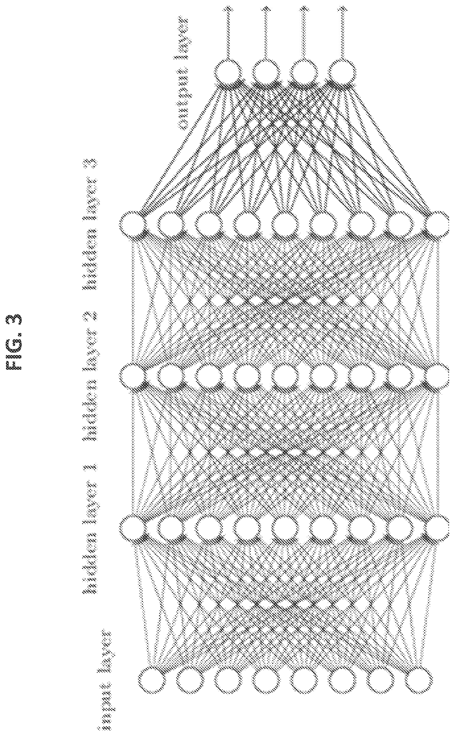

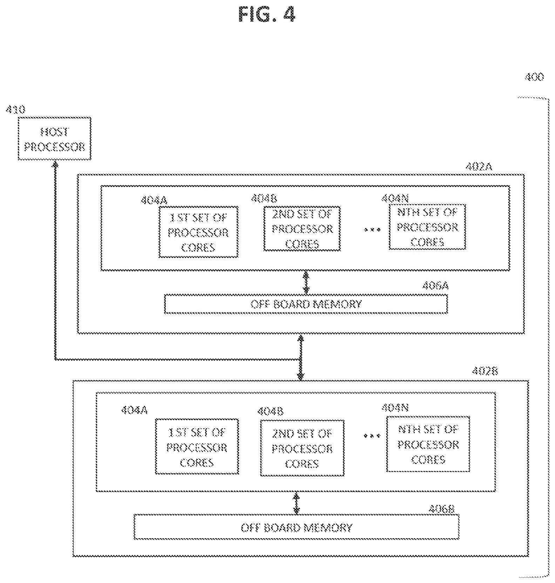

Figures 1 and 2 collectively illustrate a computer-implemented system 10 for discovery of a voice biomarker according to an illustrative embodiment of the invention. More details about certain components, e.g., components 201-207, shown in Figure 1 are shown, and described in connection with, Figure 2. Blocks 103, 105, 106, 108 in Figure 1 and blocks 201, 202, 205, 206, and 207 in Figure 2 represent various types and architectures of neural networks, such as the example neural network illustrated in Figure 3. The computations for the neural networks, including, where appropriate, forward activation and back propagation computations for training the neural network, and for other blocks of Figures 1 and 2 may be performed by a computer system, such as computer system 400 illustrated in Figure 4.

The task of the system 10 is to take an example input voice waveform and to create the biomarker 104 such that the biomarker 104 best serves to classify that example as to whether it matches a medical condition such as one of the conditions listed in Table 1 below or other medical conditions. Regardless of whether an association between voice and the given medical condition has been observed *a-priori* or not, the system 10 creates the engineered biomarker 104 that is most discriminative and least ambiguous for the condition. Biomarker 104 thus can be all of the following:
a) The target outcome of the system 10.
b) The final outcome of applying the process performed by the system 10.
c) The engineered biomarker embodiment created through the combined action of the system 10.
d) The discovered biomarker for the target condition processed by classifier 105.

The various neural networks in the system 10 may be trained by machine learning training algorithms such as gradient descent computed by estimating the gradient on mini batches of data examples with various refinements that are well known to those skilled in the art of training neural networks. The classifier 105 can be a neural network that is trained to match the target condition for the example input datum. In preferred embodiments of the invention, there are additional neural networks in 108 that are trained with other objectives, such as classifying for confusable medical conditions.

The neural networks represented by block 108 serve simultaneously as controls to impart the properties of discriminability for the engineered biomarker 104, to reduce the confusability of the biomarker 104, and to improve the reliability of the diagnosis that is the outcome of the classifier 105. Automated medical diagnosis based on biomarkers in voice is a challenging pattern recognition task with limited training data that is often only subjectively labeled. Furthermore, the cost of an error may be high; thus, reliability, discriminability and interpretability are top priorities.

The processing in the illustrative embodiment of Figure 1 begins with the computer system 400 recording or obtaining one or more input voice waveforms in block 100 from one or more persons, some of who have the condition for which the biomarker is to be discovered. These training voice recordings can be captured by a microphone(s) (not shown). The captured voice recordings can be digitized and stored in a computer database(s) for use by the system 10 (e.g., being used to train the system 10 through machine learning). The database(s) storing the training voice recordings could be co-located with the computer system 400 or the database(s) could be remote, in which case the computer system 400 can be in communication with the database(s) via an electronic data network, such as the Internet, a LAN, a WAN, etc.

In block 101, computer system 400 controls the selection and processing of one or more digital signal processing functions that are applied to the input voice waveform 100. For example, each of the DSP functions may compute a spectrogram, which is a representation the amplitude in each of a set of frequency channel as a function of time. The spectrogram may be computed, for example, by computing a fast Fourier transform (FFT) for each short time interval window centered around sequence of times ti.

In certain embodiments, blocks 102 in Figure 1 and block 201 in Figure 2 each compute one or more digital signal processing functions. The signal processing functions may vary, for example, in degree of resolution in time or frequency and in the bandwidth of the frequency measurements, in the type of transformations applied to the input voice signals, or in the type of representations or specific measurements derived from them. Blocks 102 and 201 may implement their respective digital signal processing functions with or without neural networks that emulate digital signal processing functions.

In the illustrative embodiment, in block 101, computer system 400 selects one or more variants of the digital signal processing functions to be used in block 102 and one or more variants of the digital signal processing to be used in block 201 of Figure 2.

Computer system 400 computes one or more of the digital signal processing functions to obtain a set of feature values 102 to provide as input to an encoder 103. This set of input features 102 may be represented, for example, as a set of one or more spectrograms, or as a matrix or higher dimensional tensor of values, depending on the implementation of the encoder 103.

In a preferred embodiment, encoder 103 is implemented with a neural network. For example, if the feature stack 102 is represented as a set of spectrograms or their images, encoder 103 may be a convolutional neural network. However, other suitable neural network architectures may be used for encoder 103 in various embodiments. The activation, training, and inference computations for neural network encoder 103 are performed on a computer system such as system 400.

Using encoder 103, computer system 400 transforms the input feature representation 102 into a latent space, yielding a latent feature representation 104. Computer system 400 can train the encoder 103 by gradient descent using back propagation backward through the latent feature representation 104.

Preferably, the encoder 103 is trained such that the latent feature representation 104 satisfies several criteria:
(1) The latent feature representation 104 is discriminative for the underlying medical condition it is engineered to encode. This criterion is achieved by back propagation training from the neural network 105.
(2) Information in the input representation 102 should not be lost. This criterion is achieved by back propagation from blocks 106 and 107, as will be explained below.
(3) The latent feature representation must be able to discriminate between the target condition and other medical conditions. This criterion is achieved by back propagation from neural networks in block 108, which are trained to recognize other medical conditions that might be confused with the target condition.

Computer system 400 can train the neural network 105 using back propagation for gradient descent based on training data examples of positive instances of the condition and on negative instances in which the condition does not exist. The arrow coming into the neural network 105 from the right indicates computer system 400 applying labeled data examples for supervised training, as do the other thick dashed-line arrows for the other neural networks in the system 10 shown in Figures 1 and 2. During training, computer system 400 can back propagate gradients from the neural network 105 to latent feature space 104 and then back to encoder 103. However, to satisfy criteria (2) and (3) above, as well as for additional reliability and interpretability, the latent feature space 104 and the encoder 103 also receive back propagation from additional subsystems, as described herein.

Computer system 400 sends the values of the latent variables 104 as input to decoder 106. The task of decoder 106 is to transform the latent variable representation 104 back into an output feature stack 107 that approximates the input feature stack 102 or any subcomponent of it that is known to be sufficient to reconstruct an approximation to the original voice signal. Computer system 400 trains decoder 106, for example, by back propagating an error loss function based on a measure of the difference between all of, or the chosen subcomponents of input feature stack 102 and output feature stack 107. That is, as indicated by the thick dashed-line arrow, input feature stack 102 is the target for training neural network decoder 106. No human labeling of the data is required in certain embodiments. Those skilled in the art know and understand this method of training neural network autoencoders. Subsystems 102, 103, 104, 106, and 107, if trained in isolation, would constitute an autoencoder.

Block 108 is a validator stack. It comprises, for example, one or more machine learning classifiers for other medical conditions that are different from the condition for which the biomarker 104 is being discovered/engineered. Three such classifiers V1, V2, and V3 are shown, but any number of classifiers may be used. The thirty (30) medical conditions listed in Table 1 are only a fraction of the medical conditions for which a neural network classifier in block 108 might be trained to classify in embodiments of the invention. The thick dashed-line arrows in Figure 1 indicate that the computer system 400 can train each of the neural network subblocks of block 108 using positive data examples and negative data examples of its respective medical condition. Computer system 400 back propagates gradients from each of the subblocks of block 108 to the latent feature representation 104 and then to encoder network 103.

In some embodiments, computer system 400 may transmit latent feature representation 104 to decoder 202 in Figure 2. In some embodiments computer system 400 may use the output of decoder 106 as a generated waveform 203 in Figure 2.

Figure 2 illustrates more details for certain components and subsystems of the system 10 shown in Figure 1. DSP stack 201 and decoder 202 can be similar in structure and operation to the DSP blocks 101-102 and decoder 106, respectively, both described above in the discussion of Figure 1. DSP stack 201 is a stack of digital single processing functions selected by computer system 400 from the functions in block 101 of Figure 1, applied by computer system 400 to input waveform 100 of Figure 1.

For each subblock of DSP stack 201, computer system 400 applies the corresponding digital signal processing function (which may include a neural network) to transform input voice waveform 100 to a feature stack like feature stack 102 of Figure 1. In some embodiments, computer system 400 may use the output of each subblock of block 201 as a target for training the corresponding neural network subblock of DSP neural stack 205.

For each subblock of DSP stack 201, computer system 400 uses the known input waveform 100 of Figure 1. For each subblock of DSP neural stack 205, computer system 400 uses as input the output of decoder 202, and/or a digital signal processing function (via DSP block 204) of generated waveform 203 and/or the generated waveform 203 itself. Being computed from the known input waveform 100, the subblock feature representations in block 201 are called "measured" signal processing features, whereas the feature representations in the subblocks of DSP neural stack 205 are called "predicted" signal processing features. DSP neural stack 205 will be discussed further below.

In some embodiments, computer system 400 uses the output of decoder 106 as the output of decoder 202. That is, for example, one decoder could be used for both the decoder 106 in Figure 1 and the decoder 202 in Figure 2.

In other embodiments, different decoders could be used. For example, computer system 400 may train a separate decoder 202 (i.e., separate from decoder 106) to generate outputs that are input to the DSP neural stack 205, and/or generate a waveform that is input to the DSP neural stack 205 after digital signal processing by DSP block 204. In these embodiments, computer system 400 uses input waveform 102 of Figure 1 as the target for decoder 202. In these embodiments, computer system 400 may use a different architecture for decoder 202 than for decoder 106 of Figure 1 and/or may use a different training procedure.

Computer system 400 transmits the output of decoder 202 and/or generated waveform 203 to DSP block 204. DSP block 204 is another stack of digital signal processing functions. In various embodiments, some, or all, of the digital signal processing functions in DSP block 204 may be the same as functions in DSP block 101 of Figure 1. In some embodiments, some, or all, of the digital signal processing functions in DSP block 204 may be different from those in DSP block 101 of Figure 1.

DSP neural stack 205 comprises a set of neural network subblocks corresponding to the subblocks of block 201. In various embodiments, computer system 400 may use one or both of two sources of back propagation for training the subblocks of DSP neural stack 205. For one source of back propagation, computer system 400 may use as a target for a subblock neural network in block 205 the measured feature representation of the corresponding subblock of block 201. For a second source of back propagation, computer system 400 may use back propagation from the computation of voice attributes systems 206 and 207, which are discussed below.

In some embodiments, computer system 400 computes estimated voice attributes by voice attribute systems 206 and 207. The voice attributes are attributes such as prosodic variations, the perceptual qualities of voice including but not restricted to assessments of having or being aphonic, biphonic, bleat/flutter, breathy, covered/muffled/darkened, creakiness, fluttery, glottalized, hoarse/raspy/harsh/grating, honky/nasal, jittery, rough/uneven/bumpy/unsteady, pressed, pulsed/vocal-fry, resonant/ringing/brightened, shimmery/crackly/buzzy, strained, strohbass, tremorous, twangy/sharp, ventricular, wobble/wavering/irregular, yawny, aesthenic; various objective measures of vocal fold dynamics such as degree of vocal fold closure, duration of adduction etc.; various objective measures of the sub-processes of voice production; and various other attributes such as those mentioned in the right-hand column of Table 1. For example, computer system 400 may train a separate neural network for each voice attribute. Thus, each voice attribute system 206, 207 may include a collection, or stack, of neural networks, with each such neural network trained to estimate respective voice attributes. In some embodiments, for each voice attribute, computer system 400 may use the same neural network for both voice attribute system 206 and the voice attribute system 207. In other embodiments, some, or all, of the attribute neural networks may be different in system 207 from the corresponding neural network in system 206.

In some embodiments, computer system 400 may use the voice attribute values computed in system 206 as targets for the voice attribute values in system 207. In some embodiments, the neural networks in system 206 and 207 are trained only from the target values in the voice attribute training data. In these embodiments, computer system 400 may pretrain the voice attribute neural networks in systems 206 and 207 and then back propagate the gradients from the system 206 target values back through the system 207 neural networks as a second source of back propagation to the stack of neural networks in system 205. In some embodiments, computer system 400 may use the system 206 target values in training the voice attribute neural networks in system 207 as well as back propagating the gradients from the system 206 target values to the neural networks in the DSP neural stack 205.

The inter-connected neural network subsystems of system 10 may be trained with a global, system-wide loss function, so that the various neural network subsystems are trained with a collective objective (in addition to local objectives that each neural network is trained with). The result of the simultaneously global and local objective training for the neural networks is the biomarker latent space 104, which can be used, once discovered as described herein, for many purposes, such as training machine learning classifiers to detect the condition corresponding to the biomarker in voice recordings. The system 10 may be trained with one or more voice recording waveforms of suitable duration.

Many of the subsystems of system 10, such as the encoder 103, decoder 106 and/or 202, classifier 105, validator stack subblocks 108, neural stack subblocks 201 and 205, and voice attribute systems 206, 207, may comprise a neural network. Figure 3 is a drawing of an example of a multi-layer feed-forward deep neural network. A neural network is a collection of nodes and directed arcs. The nodes in a neural network are often organized into layers. In a feed-forward neural network, the layers may be numbered from bottom to top, when diagramed as in Figure 3, with the input layer at the bottom and the output layer at the top conventionally. In other publications, the layers may be numbered from top to bottom or from left to right. No matter how the figure is drawn, feed forward activation computations proceed from lower numbered layers to higher number layers (i.e., from input to output), and the back-propagation computation proceeds from the highest numbered layers to the lower numbered layers (i.e., from output to input). Each directed arc in a layered feed-forward neural network goes from a source node in a lower numbered layer to a destination node in a higher numbered layer. The feed-forward neural network shown in Figure 3 has an input layer, an output layer, and three inner layers. An inner layer in a neural network is also called a "hidden" layer. Each directed arc is associated with a numerical value called its "weight." Typically, each node other than an input node is associated with a numerical value called its "bias." The weights and biases of a neural network are called "learned" parameters. During training, the values of the learned parameters are adjusted by the computer system 400 shown in Figure 4. Other parameters that control the training process are called hyperparameters.

**Table 1: A List of Example Health Conditions and How They Tend to Affect a Person's Voice**

| Abbreviations used for parameter type: **Ac:** Acoustic, **Ar:** Articulatory, **Ev:** Evolutionary, **Md:** Medical, **Pe:** Perceptual, **Ph:** Phonatory, **Pr:** Prosodic | | |
|---|---|---|
| **No.** | **Name of syndrome** | **Effect on voice** |
| 1 | 22q11.2 deletion syndrome | Pe: Nasal |
| 2 | Anorexia Nervosa | Md: Hyperfunctional dysphonia; Ph: Significantly short phonation times; Ac: Lower fundamental frequency F0, narrowing of the voice scale |
| 3 | Anterior operculum syndrome | Md: Aphonia |
| 4 | Bardet-Biedl syndrome | Pe: Breathy, hypernasal, high pitched, poor volume control; Pr: Slow speech; Ar: Uncoordinated oral and palatal movements, misarticulations, substitutions (particularly of the first consonant of a word), and omissions of the last syllable of a word. |
| 5 | Bilateral opercular syndrome | Md: Aphonia, anarthria, palatal palsy. |
| 6 | Bogart-Bacall syndrome | Pe: Breathy, raspy, diplophonic. |
| 7 | Cerebellar motor syndrome (CMS) | Pe: Nasal; Ar: Slurred speech; Md: Ataxic dysarthria (with scanning - explosive with staccato - feature) |
| 8 | Cri-du-chat syndrome | Pe: Breathy, raspy. |
| 9 | Down syndrome | Pe: Breathy and rough (moderately), Poor voice quality, monopitch, andmonoloudness; Ac: Higher mean F0, lower harmonics-to-noise ratio; Ph: Longer voice onset times. |
| 10 | Foix-Chavany-Marie syndrome | Pe: Breathy; Ph: Short duration of phonation (max 2s). |
| 11 | Goldenhar syndrome | Pe: Breathy, harsh, high-pitched |
| 12 | KCNK9 imprinting syndrome | Md: Dysphonia; Pe: Muffled |
| 13 | Kabuki syndrome | Md: Dysarthria (with imprecise consonants, harsh voice, hypernasality, reduced rate and stress, distorted pitch), impaired Oromotor functioning; Ev: Delayed articulation and phonology |
| 14 | Mild Cognitive Impairment syndrome (MCI) | Pe: Breathy, creaky; Ac: Strong H1, weaker H2 |
| 15 | Lujan-Fryns syndrome | Pe: Breathy, raspy |
| 16 | Nager syndrome | Ph: Altered phonetic placement; Ae: Modification of hypernasality and nasal emission; Pe: Diplophonic Ac: Low intensity |
| 17 | Ortner syndrome | Pe: Hoarse, asthenic. |
| 18 | Progressive Supranuclear Palsy and Corticobasal Syndromes | Ev: Shorter speech segments, longer pauses, higher pause rate, slower speech; Ac: Reduced fundamental frequency (f0) range |
| 19 | Pulmonary parenchymal syndrome | Ac: Increased voice fremitus |
| 20 | Rett syndrome | Pe: Breathy. Ar/Ph: proto-vowel and proto-consonant alternations produced on ingressive airstream. |
| 21 | Shprintzen-Goldberg syndrome | Md: Oral hypotonia; Pe: High-pitched soft voice, slight hypernasality |
| 22 | Simpson-Golabi-Behmel syndrome | Ar: Distorted articulation; Ac: Distorted resonances; Pr: Monotonic prosody; Ev: Fluency failures |
| 23 | Sjögren's syndrome | Md: Dysphonia Pe: Diplophonic, harsh, raspy. |
| 24 | Tay syndrome | Md: Dysphonia; Pe: Hoarse, raspy, high-pitched |
| 25 | Tourette's syndrome | Md: Dysphonia |
| 26 | Wallenberg syndrome | Pe: Breathy, moderately hyponasal |
| 27 | Werner's syndrome | Pe: Hoarse, high pitch |
| 28 | Wilson-Mikity Syndrome | Pe: Biphonic |
| 29 | Syndrome of the Jugular Foramen | Md: Partial aphonia; Pe: Hoarse |
| 30 | Worster-Drought Syndrome | Md: Severe, persistent aphonia |

Once the machine learning components of the system 10 are trained, the system 10 can be used to generate the engineered voice biomarker 104 for a subject human and to determine whether the subject human has the target medical condition (or more particularly, compute a likelihood that the subject human has the target medical condition) based on the classifications of the engineered biomarker 104 by the classifiers 105, 108. The system 10 can also determine (or compute a likelihood of) whether the subject human has voice attributes associated with the target medical condition based on the DSP neural stack 205's computation of the voice attributes 207. A voice recording of sufficient duration can be captured by a microphone. The microphone could be co-located with (and/or part of) the computer system 400, or it could be remote from the computer system 400. For example, the subject human's voice recording could be captured by the microphone and then digitized, with the digitized voice recording stored in a database (such as in the cloud), where the database is in communication with the computer system 400 via an electronic data network, such as the Internet, a LAN, a WAN, etc. The microphone may include a diaphragm that is vibrated by the sound waves from the subject human's audible utterances. The vibrations of the diaphragm can be converted to an analog signal, which can be converted to digital by an analog-to-digital converter. The digital signal can be converted to a digital audio format, lossy or lossless, such as MP3, WAV, AIFF, AAC, OGG, FLAC, ALAC, WMA, etc., for storing in the database.

Referring to Figure 1, the subject human's digitized voice recording can be processed by the applicable DSP functions at block 101. The set of measurements from the DSP functions are then encoded by the encoder 103 (after having been trained) to generate the voice biomarker 104 for the subject human. The voice biomarker 104 is discriminative for the target medical condition based on how the neural network system 10 was trained, as described herein. The classifiers 105, 108 can then compute a likelihood of whether the subject human has the target medical condition, and the DSP neural stack 205 can compute the likelihood of whether the subject human has voice attributes associated with the target medical condition.

Figure 4 is a diagram of a computer system 400 that could be used to implement the embodiments described above. The illustrated computer system 400 comprises multiple processor units 402A-B that each comprises, in the illustrated embodiment, multiple (N) sets of processor cores 404A-N. The processor cores 404A-N may include one or more digital signal processors (DSPs) that perform the digital signal processing described herein, such as for blocks 101 and 204 of Figures 1 and 2, respectively. Each processor unit 402A-B may comprise on-board memory (ROM or RAM) (not shown) and off-board memory 406A-B. The on-board memory may comprise primary, volatile and/or non-volatile storage (e.g., storage directly accessible by the processor cores 404A-N). The off-board memory 406A-B may comprise secondary, non-volatile storage (e.g., storage that is not directly accessible by the processor cores 404A-N), such as ROM, HDDs, SSD, flash, etc. The processor cores 404A-N may be CPU cores, GPU cores and/or AI accelerator cores. GPU cores operate in parallel (e.g., a general-purpose GPU (GPGPU) pipeline) and, hence, can typically process data more efficiently that a collection of CPU cores, but all the cores of a GPU execute the same code at one time. AI accelerators are a class of microprocessor designed to accelerate artificial neural networks. They typically are employed as a co-processor in a device with a host CPU 410 as well. An AI accelerator typically has tens of thousands of matrix multiplier units that operate at lower precision than a CPU core, such as 8-bit precision in an AI accelerator versus 64-bit precision in a CPU core.

In various embodiments, the different processor cores 404 may train and/or implement different networks or subnetworks or components. For example, in one embodiment, the cores of the first processor unit 402A may implement the digital signal processing functions in block 101 of Figure 1 and the second processor unit 402B may implement decoder 103. As another example, the cores of the first processor unit 402A may implement the training of the neural network encoder 103 and the neural network decoder 106, the cores of the second processing unit 402B may implement the training of the neural network classifier 105, and the cores of yet another processing unit may implement the neural network classifier V1, V2, V3, ... in the validator stack 108. One or more host processors 410 may coordinate and control the processor units 402A-B.

In other embodiments, the system 400 could be implemented with one processor unit 402. In embodiments where there are multiple processor units, the processor units could be co-located or distributed. For example, the processor units 402 may be interconnected by data networks, such as a LAN, WAN, the Internet, etc., using suitable wired and/or wireless data communication links. Data may be shared between the various processing units 402 using suitable data links, such as data buses (preferably high-speed data buses) or network links (e.g., Ethernet).

The software for the various computer system 400s described herein and other computer functions described herein may be implemented in computer software using any suitable computer programming language such as .NET, C, C++, Python, and using conventional, functional, or object-oriented techniques. Programming languages for computer software and other computer-implemented instructions may be translated into machine language by a compiler or an assembler before execution and/or may be translated directly at run time by an interpreter. Examples of assembly languages include ARM, MIPS, and x86; examples of high level languages include Ada, BASIC, C, C++, C#, COBOL, Fortran, Java, Lisp, Pascal, Object Pascal, Haskell, ML; and examples of scripting languages include Bourne script, JavaScript, Python, Ruby, Lua, PHP, and Perl.

In general aspects, therefore, the present invention is directed to a diagnostic tool that comprises a computer system. The computer system comprises: one or more processor cores; and a memory in communication with the processor cores. The memory stores software that when executed by the one or more processor cores, causes the one or more processing cores to generate, with an encoder that is trained through machine learning, a biomarker that is discriminative for a target medical condition for a subject person from a voice recording from the subject person.

In another general aspect, the present invention is directed to a method that comprises the steps of: capturing a voice recording from a subject person; and generating, with an encoder of a neural network system that is trained by a computer system through machine learning, a biomarker that is discriminative for a target medical condition for the subject person from the voice recording from the subject person.

In another general aspect, the present invention is directed to a computer system that comprises one or more processor cores; and a memory in communication with the processor cores. The memory stores software that when executed by the one or more processor cores, causes the one or more processing cores to train a neural network system to generate a biomarker that is discriminative for a target medical condition from a voice recording of a subject person.

In another general aspect, the present invention is directed to a method that comprises the step of training, through machine learning, with a computer system, a neural network system to generate a biomarker that is discriminative for a target medical condition from a voice recording of a subject person.

In various implementations, the memory further stores software that, when executed by the one or more processors, causes the one or more processors to classify, with a first classifier, the biomarker. The first classifier is trained, through machine learning, to detect the target medical condition.

In various implementations, the diagnostic tool further comprises a microphone for capturing the voice recording of the subject person.

In various implementations, the encoder is part of an autoencoder that further comprises a decoder; the decoder is trained to transform output from the encoder to an output feature stack that approximates an input feature stack for the encoder; and the encoder, the decoder, and the first classifier are trained with at least a collective objective. In various embodiments, the encoder comprises a first neural network; the decoder comprises a second neural network; and the first classifier comprises a third neural network.

In various implementations, the memory further stores software that, when executed by the one or more processors, causes the one or more processors to apply signal processing to a digitization of the voice recording of the subject person to compute a set of measurements for the voice recording of the subject person. The set of measurements can be input to the encoder and can comprise one or more spectrograms.

In various implementations, the memory further stores software that, when executed by the one or more processors, causes the one or more processors to classify the biomarker with a second classifier; and the second classifier is trained to recognize another medical condition that is confusable with the target medical condition. Also, the memory may further store software that, when executed by the one or more processors, causes the one or more processors to apply signal processing to a digitization of the voice recording of the subject person to compute a set of measurements for the voice recording of the subject person, where the set of measurements is used to compute voice attributes. In that connection, the memory may further store software that, when executed by the one or more processors, causes the one or more processors to compute one or more voice attributes from the measurements derived from the voice recording. The voice attributes may be computed by neural networks that are trained through machine learning.

In various implementations, the diagnostic tool further comprises a second decoder to derive features from the output of the encoder for predicting voice attributes. The second decoder may reconstructs a voice recording (e.g., generate a reconstructed voice recording). The memory may further store software that, when executed by the one or more processors, causes the one or more processors to compute a predicted voice feature from output of the second decoder. The memory may further store software that, when executed by the one or more processors, causes the one or more processors to apply a signal processing to the reconstructed voice recording to compute a predicted voice feature. The memory may further store software that, when executed by the one or more processors, causes the one or more processors to compute a predicted voice attribute from the predicted voice features.

In various implementations, the encoder is part of an autoencoder that further comprises a first decoder; the first decoder is trained to transform output from the encoder to an output feature stack that approximates an input feature stack for the encoder; and the diagnostic tool further comprises a second decoder that reconstructs a reconstructed voice recording. Also, the memory further stores software that, when executed by the one or more processors, causes the one or more processors to: classify, with a first classifier, the biomarker, wherein the first classifier is trained, through machine learning, to detect the target medical condition; apply a first signal processing to a digitization of the voice recording of the subject person to compute a set of measurements for the voice recording of the subject person; compute one or more computed voice attributes from the measurements computed from the voice recording; compute a predicted voice feature from output of the second decoder; and train, through machine learning, one or more machine learning components of the diagnostic tool using a mathematical objective obtained from the computed and predicted voice attributes, where the one or more machine learning components comprise one or more of the encoder, the first decoder, and the first classifier.

The examples presented herein are intended to illustrate potential and specific implementations of the present invention. It can be appreciated that the examples are intended primarily for purposes of illustration of the invention for those skilled in the art. No particular aspect or aspects of the examples are necessarily intended to limit the scope of the present invention. Further, it is to be understood that the figures and descriptions of the present invention have been simplified to illustrate elements that are relevant for a clear understanding of the present invention, while eliminating, for purposes of clarity, other elements. While various embodiments have been described herein, it should be apparent that various modifications, alterations, and adaptations to those embodiments may occur to persons skilled in the art with attainment of at least some of the advantages. The disclosed embodiments are therefore intended to include all such modifications, alterations, and adaptations without departing from the scope of the embodiments as set forth herein.

Various aspects of the invention according to the present disclosure include, but are not limited to, the aspects listed in the following numbered clauses.
Clause 1. A diagnostic tool comprising a computer system, wherein the computer system comprises: one or more processor cores; and a memory in communication with the processor cores, wherein the memory stores software that when executed by the one or more processor cores, causes the one or more processing cores to generate, with an encoder that is trained through machine learning, a biomarker that is discriminative for a target medical condition for a subject person from a voice recording from the subject person.
Clause 2. The diagnostic tool of clause 1, wherein the memory further stores software that, when executed by the one or more processors, causes the one or more processors to classify, with a first classifier, the biomarker, wherein the first classifier is trained, through machine learning, to detect the target medical condition.
Clause 3. The diagnostic tool of clause 1, further comprising a microphone for capturing the voice recording of the subject person.
Clause 4. The diagnostic tool of clause 2, wherein: the encoder is part of an autoencoder that further comprises a decoder; the decoder is trained to transform output from the encoder to an output feature stack that approximates an input feature stack for the encoder; and the encoder, the decoder, and the first classifier are trained with at least a collective objective.
Clause 5. The diagnostic tool of clause 4, wherein: the encoder comprises a first neural network; the decoder comprises a second neural network; and the first classifier comprises a third neural network.
Clause 6. The diagnostic tool of clause 2, wherein the memory further stores software that, when executed by the one or more processors, causes the one or more processors to apply signal processing to a digitization of the voice recording of the subject person to compute a set of measurements for the voice recording of the subject person, wherein the set of measurements is input to the encoder.
Clause 7. The diagnostic tool of clause 6, wherein the set of measurements comprise one or more spectrograms.
Clause 8. The diagnostic tool of clause 2, wherein: the memory further stores software that, when executed by the one or more processors, causes the one or more processors to classify the biomarker with a second classifier; and the second classifier is trained to recognize another medical condition that is confusable with the target medical condition.
Clause 9. The diagnostic tool of clause 6, wherein the memory further stores software that, when executed by the one or more processors, causes the one or more processors to apply signal processing to a digitization of the voice recording of the subject person to compute a set of measurements for the voice recording of the subject person, wherein the set of measurements is used to compute voice attributes.
Clause 10. The diagnostic tool of clause 9, wherein the memory further stores software that, when executed by the one or more processors, causes the one or more processors to compute one or more voice attributes from the measurements derived from the voice recording.
Clause 11. The diagnostic tool of clause 10, wherein the voice attributes are computed by neural networks that are trained through machine learning.
Clause 12. The diagnostic tool of clause 4, further comprising a second decoder to derive features from the output of the encoder for predicting voice attributes.
Clause 13. The diagnostic tool of clause 12, wherein the second decoder reconstructs a voice recording.
Clause 14. The diagnostic tool of clause 12, wherein the memory further stores software that, when executed by the one or more processors, causes the one or more processors to compute a predicted voice feature from output of the second decoder.
Clause 15. The diagnostic tool of clause 13, wherein the memory further stores software that, when executed by the one or more processors, causes the one or more processors to apply a signal processing to the reconstructed voice recording to compute a predicted voice feature.
Clause 16. The diagnostic tool of one of clauses 14 and 15, wherein the memory further stores software that, when executed by the one or more processors, causes the one or more processors to compute a predicted voice attribute from the predicted voice features.
Clause 17. The diagnostic tool of clause 4, wherein: the encoder is part of an autoencoder that further comprises a first decoder; the first decoder is trained to transform output from the encoder to an output feature stack that approximates an input feature stack for the encoder; the diagnostic tool further comprises a second decoder that reconstructs a reconstructed voice recording; and the memory further stores software that, when executed by the one or more processors, causes the one or more processors to: classify, with a first classifier, the biomarker, wherein the first classifier is trained, through machine learning, to detect the target medical condition; apply a first signal processing to a digitization of the voice recording of the subject person to compute a set of measurements for the voice recording of the subject person; compute one or more computed voice attributes from the measurements computed from the voice recording; compute a predicted voice feature from output of the second decoder; and train, through machine learning, one or more machine learning components of the diagnostic tool using a mathematical objective obtained from the computed and predicted voice attributes, wherein the one or more machine learning components comprise one or more of the encoder, the first decoder, and the first classifier.
Clause 18. A method comprising: capturing a voice recording from a subject person; and generating, with an encoder of a neural network system that is trained by a computer system through machine learning, a biomarker that is discriminative for a target medical condition for the subject person from the voice recording from the subject person.
Clause 19. The method of clause 18, further classifying, with a first classifier of the neural network system, the biomarker to make a determination of whether the subject person has the target medical condition, wherein the first classifier is pre-trained, through machine learning, to detect the target medical condition.
Clause 20. The method of clause 19, wherein: the encoder is part of an autoencoder that further comprises a decoder; and the method further comprises, prior to generating the biomarker, training, by a computer system that comprises one or more processor cores, the encoder, decoder and the first classifier, wherein: the decoder is trained to transform output from the encoder to an output feature stack that approximates an input feature stack for the encoder; and the encoder, the decoder, and the first classifier are trained with at least a collective objective.
Clause 21. The method of clause 20, wherein: the encoder comprises a first neural network; the decoder comprises a second neural network; and the first classifier comprises a third neural network.
Clause 22. The method of clause 20, further comprising applying, by the one or more processor cores, signal processing to a digitization of the voice recording of the subject person to compute a set of measurements for the voice recording of the subject person, wherein the set of measurements is input to the encoder.
Clause 23. The method of clause 22, wherein the set of measurements comprises one or more spectrograms.
Clause 24. The method of clause 20, further comprising: training, by the computer system, a second classifier to classify outputs of the encoder, wherein the first classifier is trained, through machine learning, to recognize another medical condition that is confusable with the target medical condition; and after training the second classifier, classifying, with the second classifier, the biomarker for the subject person to assist the determination of whether the subject person has the target medical condition.
Clause 25. The method of clause 20, further comprising applying, by the one or more processor cores, signal processing to a digitization of the voice recording of the subject person to compute a set of measurements for the voice recording of the subject person, wherein the set of measurements is used to compute voice attributes.
Clause 26. The method of clause 25, further comprising computing one or more voice attributes from the measurements computed from the voice recording.
Clause 27. The method of clause 26, wherein the one or more voice attributes are computed by neural networks that are trained through machine learning.
Clause 28. The method of clause 20, further comprising training a second decoder to derive features from the output of the encoder for predicting voice attributes.
Clause 29. The method of clause 28, further comprising, reconstructing, with the second decoder, a reconstructed voice recording.
Clause 30. The method of clause 28, further computing a predicted voice feature from an output of the second decoder.
Clause 31. The method of clause 29, further comprising applying a signal processing to the reconstructed voice recording to compute a predicted voice feature.
Clause 32. The method of one of clauses 30 and 31, further comprising computing a predicted voice attribute from the predicted voice feature.
Clause 33. The method of clause 20, wherein: the encoder is part of an autoencoder that further comprises a first decoder; the first decoder is trained to transform output from the encoder to an output feature stack that approximates an input feature stack for the encoder; the neural network system further comprises a second decoder that reconstructs a reconstructed voice recording; and the method further comprises: classifying, with a first classifier, the biomarker, wherein the first classifier is trained, through machine learning, to detect the target medical condition; applying a first signal processing to a digitization of the voice recording of the subject person to compute a set of measurements for the voice recording of the subject person; computing one or more computed voice attributes from the measurements computed from the voice recording; computing a predicted voice feature from output of the second decoder; and training, through machine learning, one or more machine learning components of the neural network system using a mathematical objective obtained from the computed and predicted voice attributes, wherein the one or more machine learning components comprise one or more of the encoder, the first decoder, and the first classifier.
Clause 34. A computer system comprising: one or more processor cores; and a memory in communication with the processor cores, wherein the memory stores software that when executed by the one or more processor cores, causes the one or more processing cores to train a neural network system to generate a biomarker that is discriminative for a target medical condition from a voice recording of a subject person.
Clause 35. The computer system of clause 34, wherein the memory further stores software that, when executed by the one or more processors, causes the one or more processors to train the neural network system to detect whether the subject person has the target medical condition based on the voice recording of the subject person.
Clause 36. The computer system of clause 35, wherein the memory further stores software that, when executed by the one or more processors, causes the one or more processors to train the neural network system by: training an autoencoder with training voice recordings, wherein the autoencoder comprises an encoder and a decoder, wherein: the encoder is trained with the training voice recordings to generate a latent feature representation from an input feature stack, wherein the input feature stack is generated from the training voice recordings; and the decoder is trained to transform the latent feature representation to an output feature stack that approximates an input feature stack for the encoder; and training a first classifier, with the training voice recordings, to detect the target medical condition from output from the encoder, wherein: the training voice recordings comprise voice recording from humans with the target medical condition and voice recording from humans without the target medical condition; and after training of the autoencoder and the first classifier, the output of the encoder from an input, digitized voice recording of the subject person can be classified by, at least in part, the first classifier to determine whether the subject person has the target medical condition.
Clause 37. The computer system of clause 36, wherein the memory further stores software that, when executed by the one or more processors, causes the one or more processors to classify, with the first classifier, the biomarker to determine whether the subject person has the target medical condition.
Clause 38. The computer system of clause 36, wherein the encoder, the decoder, and the first classifier are trained with, at least, a collective objective.
Clause 39. The computer system of clause 38, wherein: the encoder comprises a first neural network; the decoder comprises a second neural network; and the first classifier comprises a third neural network.
Clause 40. The computer system of clause 36, wherein the memory further stores software that, when executed by the one or more processors, causes the one or more processors to apply signal processing to digitizations of the training voice recordings to compute sets of measurements from the training voice recordings.
Clause 41. The computer system of clause 40, wherein the sets of measurements comprise spectrograms.
Clause 42. The computer system of clause 37, wherein the memory further stores software that, when executed by the one or more processors, causes the one or more processors to train a second classifier through machine learning, wherein the second classifier is trained to recognize another medical condition that is confusable with the target medical condition, such that after training, the output of the encoder can be classified by the second classifier to improve reliability of a classification by the first classifier.
Clause 43. The computer system of clause 42, wherein the encoder, the decoder, and the first and second classifiers are trained with, at least, a collective objective.
Clause 44. The computer system of clause 38, wherein the memory further stores software that, when executed by the one or more processors, causes the one or more processors to apply signal processing to a digitization of the voice recording of the subject person to compute a set of measurements for the voice recording of the subject person, wherein the set of measurements is used to compute voice attributes.
Clause 45. The computer system of clause 44, wherein the memory further stores software that, when executed by the one or more processors, causes the one or more processors to compute one or more voice attributes from the measurements computed from the voice recording.
Clause 46. The computer system of clause 45, wherein the voice attributes are computed by neural networks that are trained through machine learning.
Clause 47. The computer system of clause 36, further comprise a second decoder to derive features from the output of the encoder, for predicting voice attributes.
Clause 48. The computer system of clause 47, wherein the second decoder reconstructs a reconstructed voice recording.
Clause 49. The computer system of clause 47, wherein the memory further stores software that, when executed by the one or more processors, causes the one or more processors to compute a predicted voice feature from an output of the second decoder.
Clause 50. The computer system of clause 48, wherein the memory further stores software that, when executed by the one or more processors, causes the one or more processors to apply a signal processing to the reconstructed voice recording to compute a predicted voice feature.
Clause 51. The computer system of one of clauses 49 and 50, wherein the memory further stores software that, when executed by the one or more processors, causes the one or more processors to compute a predicted voice attribute from the predicted voice feature.
Clause 52. The computer system of clause 36, wherein: the encoder is part of an autoencoder that further comprises a first decoder; the first decoder is trained to transform output from the encoder to an output feature stack that approximates an input feature stack for the encoder; the neural network system further comprises a second decoder that reconstructs a reconstructed voice recording; and the memory further stores software that, when executed by the one or more processors, causes the one or more processors to: classify, with a first classifier, the biomarker, wherein the first classifier is trained, through machine learning, to detect the target medical condition; apply a first signal processing to a digitization of the voice recording of the subject person to compute a set of measurements for the voice recording of the subject person; compute one or more computed voice attributes from the measurements computed from the voice recording; compute a predicted voice feature from output of the second decoder; and train, through machine learning, one or more machine learning components of the neural network system using a mathematical objective obtained from the computed and predicted voice attributes, wherein the one or more machine learning components comprise one or more of the encoder, the first decoder, and the first classifier.
Clause 53. A method comprising training, through machine learning, with a computer system, a neural network system to generate a biomarker that is discriminative for a target medical condition from a voice recording of a subject person.
Clause 54. The method of clause 53, further comprising training, by the computer system, the neural network system to detect whether the subject person has the target medical condition based on the voice recording of the subject person.
Clause 55. The method of clause 54, wherein training the neural network system comprises: training an autoencoder with training voice recordings, wherein the autoencoder comprises an encoder and a decoder, wherein: the encoder is trained with the training voice recordings to generate a latent feature representation from an input feature stack, wherein the input feature stack is generated from the training voice recordings; and the decoder is trained to transform the latent feature representation to an output feature stack that approximates an input feature stack for the encoder; and training a first classifier, with the training voice recordings, to detect the target medical condition from output from the encoder, wherein: the training voice recordings comprise voice recording from humans with the target medical condition and voice recording from humans without the target medical condition; and after training of the autoencoder and the first classifier, the output of the encoder from an input, digitized voice recording of the subject person can be classified by, at least in part, the first classifier to determine whether the subject person has the target medical condition.
Clause 56. The method of clause 55, further comprising, after training the neural network system, classifying the biomarker with the first classifier to make a determination of whether the subject person has the target medical condition.
Clause 57. The method of clause 55, wherein training the encoder, the decoder, and the first classifier comprises training the encoder, the decoder, and the first classifier, with, at least, a collective objective.
Clause 58. The method of clause 57, wherein: the encoder comprises a first neural network; the decoder comprises a second neural network; and the first classifier comprises a third neural network.
Clause 59. The method of clause 55, further comprising applying, by the computer system, signal processing to digitizations of the training voice recordings to compute sets of measurements from the training voice recordings.
Clause 60. The method of clause 59, wherein the sets of measurements comprise spectrograms.
Clause 61. The method of clause 56, further comprising training, by the computer system, a second classifier through machine learning, wherein the second classifier is trained to recognize another medical condition that is confusable with the target medical condition, such that after training, the output of the encoder can be classified by the second classifier to improve reliability of a classification by the first classifier.
Clause 62. The method of clause 61, wherein training the encoder, the decoder, and the first and second classifiers comprises training the encoder, with, at least, a collective objective.
Clause 63. The method of clause 59, further comprising applying, by the computer system, signal processing to a digitization of the voice recording of the subject person to compute a set of measurements for the voice recording of the subject person, wherein the set of measurements is used to compute voice attributes.
Clause 64. The method of clause 63, further comprising computing, by the computer system, one or more voice attributes from the measurements computed from the voice recording.
Clause 65. The method of clause 64, further comprising training through machine learning neural networks that compute the one or more voice attributes.
Clause 66. The method of clause 55, further comprising training a second decoder to derive features from the output of the encoder for predicting voice attributes.
Clause 67. The method of clause 66, wherein training the second decoder comprises training the second decoder to reconstruct a reconstructed voice recording.
Clause 68. The method of clause 66, further comprising computing, by the computer system, a predicted voice feature from an output of the second decoder.
Clause 69. The method of clause 67, further comprising applying, by the computer system, a signal processing to the reconstructed voice recording to compute a predicted voice feature.
Clause 70. The method of one of clauses 68 and 69, further comprising training through machine learning, neural networks that compute a predicted voice attribute from the predicted voice features.
Clause 71. The method of clause 55, wherein: the encoder is part of an autoencoder that further comprises a first decoder; the first decoder is trained to transform output from the encoder to an output feature stack that approximates an input feature stack for the encoder; the neural network system further comprises a second decoder that reconstructs a reconstructed voice recording; and the method further comprises: classifying, with a first classifier, the biomarker, wherein the first classifier is trained, through machine learning, to detect the target medical condition; applying a first signal processing to a digitization of the voice recording of the subject person to compute a set of measurements for the voice recording of the subject person; computing one or more computed voice attributes from the measurements computed from the voice recording; computing a predicted voice feature from output of the second decoder; and training, through machine learning, one or more machine learning components of the neural network system using a mathematical objective obtained from the computed and predicted voice attributes, wherein the one or more machine learning components comprise one or more of the encoder, the first decoder, and the first classifier.

## Claims

**1.** A computer system comprising:
one or more processor cores; and
a memory in communication with the processor cores, wherein the memory stores software that when executed by the one or more processor cores, causes the one or more processing cores to train a neural network system to generate a biomarker that is discriminative for a target medical condition from a voice recording of a subject person, wherein the memory further stores software that, when executed by the one or more processors, causes the one or more processors to train the neural network system to detect whether the subject person has the target medical condition based on the voice recording of the subject person, wherein the memory further stores software that, when executed by the one or more processors, causes the one or more processors to train the neural network system by:
training an autoencoder with training voice recordings, wherein the autoencoder comprises an encoder and a decoder, wherein:
the encoder is trained with the training voice recordings to generate a latent feature representation from an input feature stack, wherein the input feature stack is generated from the training voice recordings; and
the decoder is trained to transform the latent feature representation to an output feature stack that approximates an input feature stack for the encoder; and
training a first classifier, with the training voice recordings, to detect the target medical condition from output from the encoder,
wherein:
the training voice recordings comprise voice recording from humans with the target medical condition and voice recording from humans without the target medical condition; and
after training of the autoencoder and the first classifier, the output of the encoder from an input, digitized voice recording of the subject person can be classified by, at least in part, the first classifier to determine whether the subject person has the target medical condition.

**2.** The computer system of claim 1, wherein the memory further stores software that, when executed by the one or more processors, causes the one or more processors to classify, with the first classifier, the biomarker to determine whether the subject person has the target medical condition.

**3.** The computer system of claim 1, wherein the memory further stores software that, when executed by the one or more processors, causes the one or more processors to apply signal processing to digitizations of the training voice recordings to compute sets of measurements from the training voice recordings, optionally, wherein the sets of measurements comprise spectrograms.

**4.** The computer system of claim 2, wherein the memory further stores software that, when executed by the one or more processors, causes the one or more processors to train a second classifier through machine learning, wherein the second classifier is trained to recognize another medical condition that is confusable with the target medical condition, such that after training, the output of the encoder can be classified by the second classifier to improve reliability of a classification by the first classifier.

**5.** The computer system of claim 1 or claim 4, wherein the encoder, the decoder, and the first classifier and/or the second classifier are trained with, at least, a collective objective.

**6.** The computer system of claim 5, wherein the memory further stores software that, when executed by the one or more processors, causes the one or more processors to apply signal processing to a digitization of the voice recording of the subject person to compute a set of measurements for the voice recording of the subject person, wherein the set of measurements is used to compute voice attributes.

**7.** The computer system of claim 6, wherein the memory further stores software that, when executed by the one or more processors, causes the one or more processors to compute one or more voice attributes from the measurements computed from the voice recording, optionally, wherein the voice attributes are computed by neural networks that are trained through machine learning.

**8.** The computer system of claim 1, further comprises a second decoder to derive features from the output of the encoder, for predicting voice attributes9. The computer system of claim 8, wherein
the second decoder reconstructs a reconstructed voice recording; and/or
the memory further stores software that, when executed by the one or more processors, causes the one or more processors to compute a predicted voice feature from an output of the second decoder,
wherein, when the second decoder reconstructs the reconstructed voice recording, the memory further stores software that, when executed by the one or more processors, causes the one or more processors to apply a signal processing to the reconstructed voice recording to compute a predicted voice feature, and
wherein the memory further stores software that, when executed by the one or more processors, causes the one or more processors to compute a predicted voice attribute from the predicted voice feature.

**10.** A diagnostic tool comprising a computer system, wherein the computer system comprises:
one or more processor cores; and
a memory in communication with the processor cores, wherein the memory stores software that when executed by the one or more processor cores, causes the one or more processing cores to generate, with an encoder that is trained through machine learning, a biomarker that is discriminative for a target medical condition for a subject person from a voice recording from the subject person, wherein the memory further stores software that, when executed by the one or more processors, causes the one or more processors to classify, with a first classifier, the biomarker, wherein the first classifier is trained, through machine learning, to detect the target medical condition, wherein:
the encoder is part of an autoencoder that further comprises a decoder;
the decoder is trained to transform output from the encoder to an output feature stack that approximates an input feature stack for the encoder; and
the encoder, the decoder, and the first classifier are trained with at least a collective objective.

**11.** A method comprising:
capturing a voice recording from a subject person; and
generating, with an encoder of a neural network system that is trained by a computer system through machine learning, a biomarker that is discriminative for a target medical condition for the subject person from the voice recording from the subject person, further classifying, with a first classifier of the neural network system, the biomarker to make a determination of whether the subject person has the target medical condition, wherein the first classifier is pre-trained, through machine learning, to detect the target medical condition, wherein:
the encoder is part of an autoencoder that further comprises a decoder; and
the method further comprises, prior to generating the biomarker, training, by a computer system that comprises one or more processor cores, the encoder, decoder and the first classifier, wherein:
the decoder is trained to transform output from the encoder to an output feature stack that approximates an input feature stack for the encoder; and
the encoder, the decoder, and the first classifier are trained with at least a collective objective.

**12.** The method of claim 11,
further comprising applying, by the one or more processor cores, signal processing to a digitization of the voice recording of the subject person to compute a set of measurements for the voice recording of the subject person, wherein the set of measurements is input to the encoder, optionally, wherein the set of measurements comprises one or more spectrograms.

**13.** The method of claim 11, further comprising:
training, by the computer system, a second classifier to classify outputs of the encoder, wherein the first classifier is trained, through machine learning, to recognize another medical condition that is confusable with the target medical condition; and
after training the second classifier, classifying, with the second classifier, the biomarker for the subject person to assist the determination of whether the subject person has the target medical condition, or,
further comprising applying, by the one or more processor cores, signal processing to a digitization of the voice recording of the subject person to compute a set of measurements for the voice recording of the subject person, wherein the set of measurements is used to compute voice attributes.

**14.** The method of claim 13, further comprising computing one or more voice attributes from the measurements computed from the voice recording, optionally, wherein the one or more voice attributes are computed by neural networks that are trained through machine learning.

**15.** The method of claim 11, further comprising training a second decoder to derive features from the output of the encoder for predicting voice attributes; and/or
further comprising, reconstructing, with the second decoder, a reconstructed voice recording; and/or
, further computing a predicted voice feature from an output of the second decoder; and/or
further comprising applying a signal processing to the reconstructed voice recording to compute a predicted voice feature; and/or
further comprising computing a predicted voice attribute from the predicted voice feature.
